# EUROPÄISCHE PATENTSCHRIFT

(11) **EP 3 648 812 B1**
(45) Veröffentlichungstag und Bekanntmachung des Hinweises auf die Patenterteilung: **06.11.2024**
(21) Anmeldenummer: 18740527.9
(22) Anmeldetag: 03.07.2018
(51) Int. Cl.: A61M 1/14, F04B 43/067, F04B 49/12, F04B 49/14, F04B 49/16

(54) **PUMPSYSTEM UND DIALYSEGERÄT**
PUMPING SYSTEM AND DIALYSIS APPARATUS
SYSTÈME DE POMPAGE ET APPAREIL DE DIALYSE

(30) Priorität: 04.07.2017 DE 102017114895
(43) Veröffentlichungstag der Anmeldung: 13.05.2020
(73) Patentinhaber: Fresenius Medical Care Deutschland GmbH, 61352 Bad Homburg (DE)
(72) Erfinder: HEDMANN, Frank, 97332 Volkach (DE); SEBESTA, Sven, 91353 Hausen (DE); HOCHREIN, Torsten, 97478 Eschenau (Knetzgau) (DE)
(74) Vertreter: Herrmann, Uwe
(86) Internationale Anmeldenummer: PCT/EP2018/068005
(87) Internationale Veröffentlichungsnummer: WO 2019/007992

(56) Entgegenhaltungen:
- WO-A1-2013/045711
- WO-A2-2016/059614
- CH-A- 333 650
- DE-A1- 19 742 632
- DE-A1- 19 919 572
- US-A- 3 204 631
- US-A- 4 710 166
- US-A1- 2003 136 189
- US-A1- 2013 006 171
- US-B1- 6 905 479
- US-B2- 7 935 074

## Beschreibung

Die Erfindung betrifft ein Pumpsystem zur Einstellung eines vorzugsweise kontinuierlichen Volumenstroms von Dialyselösung in einer Dialysemaschine, sowie eine Dialysemaschine, vorzugsweise eine Peritonealdialysemaschine, mit einem solchen Pumpsystem.

Aus dem Stand der Technik sind unterschiedliche Dialyseverfahren bekannt. Bei der automatischen Peritonealdialyse steuert eine Peritonealdialysemaschine die Einführung der frischen Dialyselösung in die Bauchhöhle und das Auslassen der verbrauchten Dialyselösung aus der Bauchhöhle.

Dokument US6905479 offenbart eine Pumpkartusche mit integriertem Filter und Verfahren zum Filtern einer Flüssigkeit mit der Kartusche.

Zur Erzeugung des Flusses von Dialyselösung können gravimetrische Geräte eingesetzt werden, d.h. solche bei denen der Fluss durch die Schwerkraft bewirkt wird sowie auch solche Geräte, bei denen zur Förderung der Dialyselösung Pumpen eingesetzt werden.

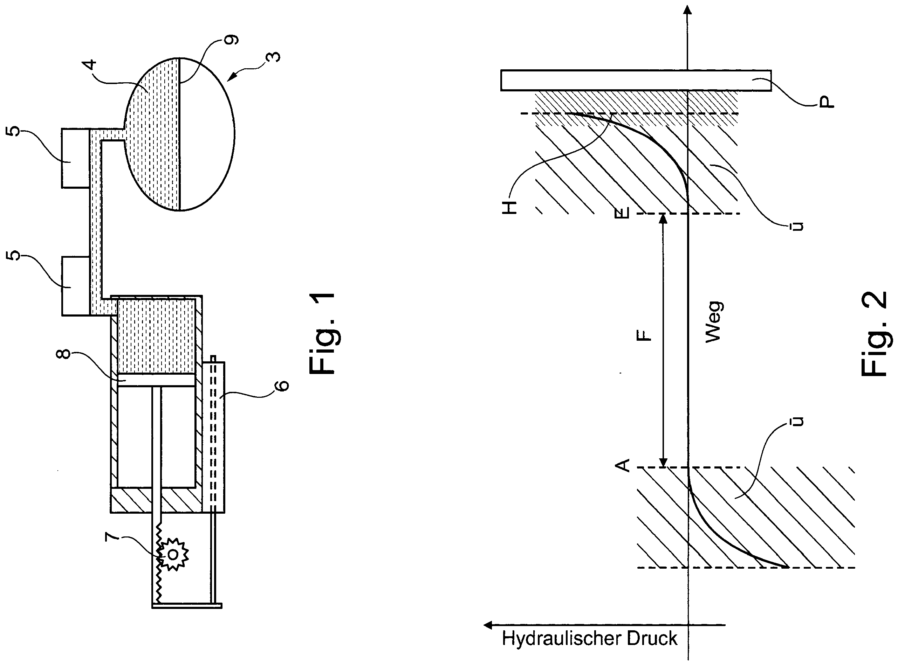

Dabei kommen häufig Kolbenpumpen zum Einsatz. Wie dies aus Figur 1 ersichtlich ist, umfasst ein bekanntes Pumpsystem einen in einem zylindrischen Raum angeordneten Kolben 8, der mit einer Kolbenstange verbunden ist, die ihrerseits durch ein motorisch betriebenes Ritzel 7 angetrieben wird. Das Ritzel 7 wirkt mit dem Zahnstangenabschnitt der Kolbenstange zusammen, so dass die Kolbenstange je nach Drehrichtung des Ritzels gemäß Figur 1 nach rechts oder links bewegt wird, d.h. in Förderrichtung oder in Ansaugrichtung.

Die Kolben 8 steht mit einem gestrichelt kennzeichneten Arbeitsfluid in Kontakt, das den Arbeitsraum des zylindrischen Raums auf der Seite des Kolbens, die von der Kolbenstange abgewandt ist, sowie einen sich daran anschließenden Leitungsabschnitt und schließlich eine Kammer 4 ausfüllt, die auf einer Seite durch eine Membran 9 verschlossen ist.

Die Membran 9 liegt an einer flexiblen Folie bzw. Membran der als Einwegartikel ausgeführten Kassette an. Die Membran 9 kann dabei z. B. aus Silikon gefertigt sein. Durch Anlegen eines Überdrucks in der Kammer 4 wird die Membran 9 und mit dieser die flexible Folie in die Pumpkammer 3 der Kassette hineingedrückt. Wird hingegen ein Unterdruck an die Kammer 4 angelegt, indem der Kolben 8 nach links bewegt wird, wird die Membran 9 dagegen in die Kammer 4 hineingezogen. Aufgrund des Unterdrucks zwischen der flexiblen Folie und der Membran folgt die flexible Folie dieser Bewegung, so dass sich das Volumen der Pumpkammer 3 vergrößert.

Über den Wegaufnehmer 6 kann die Bewegung des Kolbens aufgenommen werden. Hierdurch kann bestimmt werden, wieviel Hydraulikflüssigkeit in die Kammer 4 hineingepresst bzw. wieviel Hydraulikflüssigkeit aus dieser entnommen wurde. Weiterhin sind Drucksensoren 5 an der Hydraulik vorgesehen, welche den Druck in dem Hydrauliksystem messen. Diese ermöglichen zum einen eine Funktionsüberprüfung der Hydraulik, da die Daten der Drucksensoren mit denen des Wegaufnehmers 6 verglichen werden können und hierdurch die Dichtigkeit des Hydrauliksystems überprüft werden kann.

Zudem ermöglichen die Drucksensoren eine Bestimmung des Drucks in der Pumpkammer 3 der Kassette. Wird die Hydraulikpumpe nicht bewegt, so stellt sich ein Druckgleichgewicht zwischen der Kammer 4 und der Pumpkammer 3 ein. Der Druck der Hydraulikflüssigkeit entspricht dann dem Druck in der Pumpenkammer 3. Pumpen von Peritonealdialysegeräten fördern im Einlauf der Dialyselösung in den Bauchraum des Patienten Volumina im Bereich von 25 ml bis 3500 ml. Bauartbedingt ist mit Toleranzen von bis zu 10 % zu rechnen, was bei einem minimalen Einlaufvolumen von 25 ml ggf. zu einer Überfüllung von 2,5 ml führt. Abgesehen davon kann ein Fehler der Leistungstreiber der Pumpe dazu führen, das trotz einer anderweitigen Verschreibung eine komplette Kammer verabreicht wurde, d.h. der maximal mögliche Kolbenweg durchlaufen wurde und somit ein maximal mögliches Volumen verabreicht wurde. Wurde ein vergleichsweise kleines Einlaufvolumen verschrieben, kann dies zu einer erheblichen Überfüllung des Patienten führen. Dies wiederum kann eine Patientengefährdung zur Folge haben, die durch Maßnahmen der funktionalen Sicherheit nicht abgefangen werden kann.

Der vorliegenden Erfindung liegt somit die Aufgabe zugrunde, ein Pumpsystem der eingangs genannten Art dahingehend weiterzubilden, dass die Wahrscheinlichkeit für die Überfüllung eines Patienten gegenüber bekannten System verringert ist. Diese Aufgabe wird durch ein Pumpsystem mit den Merkmalen des Anspruchs 1 gelöst.

Danach ist vorgesehen, dass das Pumpsystem Einstellmittel aufweist, durch die das Fördervolumen der Kolbenpumpe pro Kolbenhub verringerbar und vergrößerbar ist, wobei die Einstellmittel einen Kolbenanschlag zur Verringerung und Vergrößerung des Kolbenhubs und/oder Mittel zur Verringerung und Vergrößerung der Menge des Arbeitsfluids umfassen, wobei diese Mittel einen Vorratsbehälter zur Aufnahme des Arbeitsfluids und ein Ventil umfassen, die es ermöglichen, dass das Volumen des Arbeitsfluids verringert und vergrößert wird.

In einer Ausführungsform liegt der Erfindung somit der Gedanke zugrunde, den Verfahrweg des Kolbens der Pumpe und somit das Fördervolumen pro Kolbenhub durch einen mechanischen Anschlag zu begrenzen. Der Anschlag befindet sich vorzugsweise am Kammerende, d.h. am ausstoßseitigen Ende des Arbeitsraums, in dem der Kolben beweglich aufgenommen ist. Die Begrenzung des Verfahrweges des Kolbens bringt den Vorteil mit sich, dass auch kleine Volumina mit hoher Genauigkeit gefördert werden können.

Durch diese Maßnahme wird das pro Kolbenhub förderbare Volumen an Dialysierflüssigkeit begrenzt, so dass selbst bei einem Fehler in der Leistungselektronik des Pumpsystems keine Überfüllung des Patienten eintritt.

Der Anschlag ist an unterschiedlichen Stellen positionierbar und vorzugsweise auch so einstellbar oder entfernbar, dass der Kolben auch wieder den kompletten, d.h. maximalen Verfahrweg durchlaufen kann, um ein maximales Fördervolumen pro Kolbenhub fördern zu können.

In einer weiteren Ausführungsform ist es vorgesehen, dass die Einstellmittel durch Mittel zur Verringerung des Volumens des Arbeitsfluids gebildet werden, das der Kolben in Bewegung setzt, um die Membran auszulenken und somit die Förderung der Dialyselösung zu bewirken. Durch diese Mittel kann somit das Volumen des Arbeitsfluids verringert werden, beispielsweise indem ein Teil des Arbeitsfluids in einen Vorratsbehälter abgeleitet wird. Wird wieder mehr Arbeitsfluid benötigt, kann dessen Volumen entsprechend wieder erhöht werden.

Da die Menge bzw. das Volumen des Arbeitsfluids in dem Pumpsystem das maximale Fördervolumen bestimmt, kann durch Anpassung dieser Menge bzw. dieses Volumens das Fördervolumen pro Kolbenhub variiert werden.

Grundsätzlich kann die Bestimmung der Menge bzw. des Volumens aus dem verschrieben Einlaufvolumen der Dialyselösung abgeleitet werden. So ist es möglich, das Volumen des Arbeitsfluids für eine komplette Behandlung zu verkleinern, so dass pro Kolbenhub dauerhaft nur eine entsprechend verringerte Menge an Dialyselösung gefördert wird oder dieses Volumen nur für den oder die letzten Pumpenhübe eines Einlaufs zu konfigurieren.

Diese Vorgehensweise ist nicht nur bei der Veränderung des Volumens des Arbeitsfluids denkbar, sondern auch bei den weiteren Alternativen der Ausführung der Einstellmittel.

In einer weiteren Ausführungsform ist vorgesehen, dass die Einstellmittel Mittel zur Verringerung des Volumens der Förderkammer bilden, die mit dem Fördermittel des Pumpsystems zusammenwirkt und die die zu fördernde Dialyselösung enthält. Die Förderkammer ist vorzugsweise Bestandteil eines Disposables, d.h. eines Einwegartikels, der mit dem Dialysegerät derart zusammenwirkt, dass die Membran oder ein sonstiges Förderorgan des Pumpensystems mit einer Membran zusammenwirkt, die die Förderkammer des Disposables begrenzt. Die Membran des Pumpsystems wirkt somit mit der Membran des Dispoables zusammen.

Wird die Membran des Pumpsystems durch einen Kolbenhub ausgelenkt, erfolgt eine entsprechende Bewegung der Membran der Förderkammer und somit eine entsprechende Förderung der Dialyselösung.

Eine Einschränkung des Pumpenhubs ist somit auch dadurch möglich, dass eine räumliche Beschränkung der Förderkammer der Kassette, d.h. des Disposables vorgenommen wird. Dies kann beispielsweise dadurch erreicht werden, dass gegenüber einem Ausgangszustand, in dem höhere Förderraten gewünscht sind, eine Kassette mit einer größeren Wandstärke oder mit einem anderweitig reduzierten Volumen zum Einsatz kommt.

Letztlich kann somit auch durch eine Verkleinerung des Fördervolumens der Kassette bzw. der Förderkammer eine Verringerung des Kolbenhubs erreicht werden, was ebenfalls die Genauigkeit der Förderung der Dialyselösung verbessert. Somit muss nicht zwingend ein mechanischer Anschlag im Pumpensystem vorgesehen werden. Eine Verringerung des Kolbenhubs lässt sich auch durch eine Verringerung des Fördervolumens der Kammer realisieren, mit der die Membran des Pumpsystems zusammenwirkt.

Das Pumpsystem kann eine beliebige Kolbenpumpe aufweisen, beispielsweise eine hydraulische Kolbenpumpe oder auch eine pneumatische Kolbenpumpe. Somit kann das Arbeitsfluid eine Flüssigkeit oder ein Gas sein.

In einer weiteren Ausgestaltung der Erfindung sind Eingabemittel vorgesehen, durch die das gesamte Soll-Fördervolumen oder das Soll-Fördervolumen ab einem bestimmten Zeitpunkt oder das Soll-Fördervolumen pro Kolbenhub eingebbar ist, und dass die Einstellmittel mit dem Eingabemitteln derart in Verbindung stehen, dass die Einstellmittel in Abhängigkeit der in die Eingabemittel eingegebenen Werte eingestellt werden.

So ist es beispielsweise denkbar, dass der Nutzer des Pumpsystems ein Soll-Fördervolumen, z.B. 5 I Dialyselösung vorgibt. Die Eingabemittel können beispielsweise durch einen Touchscreen oder eine Tastatur oder dergleichen gebildet werden. Auch ist es denkbar, dass der Nutzer des Pumpsystems erst ab einem bestimmten Zeitpunkt das noch zu verabreichende Soll-Fördervolumen eingibt. Ist das Gesamtvolumen der zu verabreichenden Dialyselösung beispielsweise 5 I kann vorgesehen sein, dass nach der Verabreichung von 4 I nutzerseitig eingegeben wird, dass noch 1 I zu verabreichen ist.

In einer Ausführungsform ist denkbar, dass der Nutzer das Soll-Fördervolumen pro Kolbenhub eingibt.

In Abhängigkeit von den eingegeben Daten bestimmt das Pumpsystem das Fördervolumen pro Kolbenhub und stellt dies mit Hilfe der Einstellmittel ein. Denkbar ist es beispielsweise, dass die ersten vier Liter mit einem vergleichsweise großen Fördervolumen pro Kolbenhub verabreicht werden und der verbleibende letzte Liter mit einem demgegenüber geringeren Fördervolumen pro Kolbenhub. Diese Einstellung kann durch das Pumpsystem bzw. das Dialysegerät oder auch manuell vorgenommen werden.

Grundsätzlich gilt, dass das Pumpsystem auch so ausgeführt sein kann, dass selbständig bestimmt wird, welches Fördervolumen pro Kolbenhub eingestellt wird. Das Pumpsystem kann diese Einstellung beispielsweise in Abhängigkeit von der Behandlungszeit, von dem insgesamt zu verabreichenden Volumen an Dialyselösung, dem bereits verabreichten oder dem noch zu verabreichenden Volumen an Dialyselösung etc. einstellen. In diesem Fall kann auf die oben genannten durch einen Nutzer zu betätigenden Eingabemittel verzichtet werden.

Die Einstellmittel bzw. das Pumpsystem können derart konfiguriert sein, dass das Fördervolumen pro Kolbenhub über die gesamte Förderdauer konstant ist. Ist das insgesamt zu verabreichende Fördervolumen der Dialyselösung vergleichsweise gering, ist es sinnvoll, ein möglichst kleines Fördervolumen pro Kolbenhub einzustellen.

Bevorzugt ist es, wenn die Einstellmittel bzw. die Pumpmittel derart konfiguriert sind, dass das Fördervolumen pro Kolbenhub veränderlich ist und insbesondere zum Ende des Fördervorgangs geringer ist als zu Beginn des Fördervorgangs. So ist es möglich zu Beginn der Verabreichung von Dialyselösung vergleichsweise große Fördervolumina zu verabreichen, was eine Zeitersparnis darstellt und gegen Ende des Verabreichungsvorgangs, wenn nur noch ein verhältnismäßig geringes Restvolumen zum Patienten zu fördern ist, das Fördervolumen pro Kolbenhub zu verringern bzw. auf einen kleineren Wert einzustellen, was den oben genannten Vorteil einer präziseren Förderung und der Vermeidung einer Überfüllung des Patienten hat.

Denkbar ist es weiterhin, dass die Einstellmittel derart ausgebildet sind, dass die Menge des Arbeitsfluids verringerbar ist, wobei vorgesehen ist, dass die Menge des Arbeitsfluids ausgehend von einem Ausgangszustand derart verringert wird, dass der Einfluss der Membranspannung auf den Patientendruck und/oder auf den Druck des Arbeitsfluids geringer ist als im Ausgangszustand. Je geringer die Membranspannung ist, desto geringer ist deren Einfluss auf den gemessenen Druck im Arbeitsfluid. Im Gleichgewichtszustand, wenn keine Förderung durch das Pumpsystem stattfindet, entspricht der gemesse Druck im Arbeitsfluid dem in der Förderkammer, so das auf den Patientendruck, d.h. auf den Druck in der dem Patienten verabreichten Dialyselösung geschlossen werden kann.

Somit kann beispielsweis die Einstellung der Menge bzw. des Volumens des Arbeitsfluids genutzt werden, um das Verhalten der Membran aus dem Arbeitsbereich der Kolbenpumpe in den Anschlagsbereich zu verschieben. Somit kann die Erfassung des Patientendrucks positiv beeinflusst und Verfälschungen durch die Membranspannung entfernt werden.

Vorzugsweise weist die Pumpe des Pumpsystems einen Kolbenraum auf, der über einen Schlauch mit einer Kammer verbunden ist, die durch die Membran begrenzt wird. Die Membran wirkt ihrerseits vorzugsweise auf eine als Disposable ausgeführte Kassette bzw. auf deren Förderkammer ein, wodurch das Fördervolumen zum Patienten geleitet wird.

Um einen kontinuierlichen oder weitgehend kontinuierlichen Fluss von Dialyselösung zu erzielen, weist das Pumpsystem vorzugsweise zwei Kolbenpumpen auf, die versetzt arbeiten.

An dieser Stelle wird darauf hingewiesen, dass die Begriffe "ein" und "eine" nicht zwingend auf genau eines der Elemente verweisen, wenngleich dies eine mögliche Ausführung darstellt, sondern auch eine Mehrzahl der Elemente bezeichnen können. Ebenso schließt die Verwendung des Plurals auch das Vorhandensein des fraglichen Elementes in der Einzahl ein und umgekehrt umfasst der Singular auch mehrere der fraglichen Elemente.

Die vorliegende Erfindung betrifft des Weiteren ein Dialysegerät, insbesondere ein Peritonealdialysegerät, wobei das Dialysegerät ein Pumpsystem nach einem der Ansprüche 1 bis 8 aufweist.

Des Weiteren wird ein Verfahren zum Betrieb eines Pumpsystems offenbart, wobei zum Zwecke der Erhöhung der Fördergenauigkeit der Kolbenpumpe das Fördervolumen pro Kolbenhub verringert wird, indem ein Kolbenanschlag derart verändert wird, dass der Kolbenhub verringert wird und/oder indem die Menge des Arbeitsfluids verringert wird und/oder indem das Volumen der Förderkammer, die mit dem Fördermittel zusammenwirkt und die zu fördernde Dialyselösung enthält, verringert wird.

Bevorzugt ist es, wenn in Eingabemittel das gesamte Soll-Fördervolumen oder das Soll-Fördervolumen ab einem bestimmten Zeitpunkt oder das Soll-Fördervolumen pro Kolbenhub eingegeben wird und dass die Verkleinerung des Fördervolumens in Abhängigkeit von dem oder den in die Eingabemittel eingegebenen Werten verkleinert wird. Wie oben ausgeführt, ist es auch denkbar, dass auf solche Eingabemittel verzichtet wird, und das Pumpsystem bzw. das Dialysegerät selbst bestimmt, ab welchem Zeitpunkt welches Fördervolumen pro Kolbenhub eingestellt wird.

Das Fördervolumen pro Kolbenhub kann über die gesamte Förderdauer konstant gehalten werden oder verändert werden, wobei vorzugsweise vorgesehen ist, dass das Fördervolumen pro Kolbenhub zum Ende des Fördervorgangs geringer ist als zu Beginn des Fördervorgangs, um die Genauigkeit bei der Förderung des dann vergleichsweise geringen Rest-Fördervolumens so groß wie möglich zu gestalten. Weitere Einzelheiten und Vorteile der Erfindung werden anhand eines in der Zeichnung dargestellten Ausführungsbeispiels näher erläutert.

Es zeigen:
- Figur 1:: eine schematische Ansicht eines Pumpsystems,
- Figur 2:: ein Diagramm zur Darstellung des Druckverlaufs im Arbeitsfluid und
- Figur 3:: eine weitere Prinzipdarstellung des Pumpsystems eines Peritonealdialysegerät.

Figur 1 zeigt ein Pumpsystem, das aus dem Stand der Technik bekannt ist, jedoch auch im Rahmen der vorliegenden Erfindung zur Anwendung kommen kann, d.h. das auch erfindungsgemäß ist. Auf die obigen Ausführungen zu Figur 1 wird daher verwiesen.

Die Verringerung des Fördervolumens pro Kolbenhub kann durch einen nicht dargestellten mechanischen Kolbenanschlag vorzugsweise am Boden, d.h. im Bereich des Kammerendes, in dem sich der Kolben bewegt, erfolgen. Eine Alternative besteht in der Verringerung des Volumens der Hydraulikflüssigkeit bzw. des sonstigen Arbeitsfluids, das sich zwischen dem Kolben und der Membran erstreckt und die Bewegung des Kolbens auf die Membran überträgt. Um das Volumen zu verändern, kann ein Vorratsbehälter und ein Ventil vorgesehen werden, die es ermöglichen, dass das Arbeitsmedium in den Hydraulikkreis etc. aufgenommen oder aus diesem abgezogen wird. Diese Komponenten werden derzeit genutzt, um das Arbeitsfluid zu entgasen und somit einen maximalen, bestmöglichen Verfahrweg des Kolbens zu erreichen.

Grundsätzlich ist die Erfindung nicht darauf beschränkt, eine Verringerung des pro Kolbenhub geförderten Volumens vorzunehmen, sondern es kann natürlich auch wieder eine Erhöhung dieses Volumens vorgenommen werden, sofern dies gewünscht ist.

Eine weitere Alternative der Einstellung besteht in der Verringerung des Volumens einer Kassette etc., die mit der Membran des Pumpsystems zusammenwirkt und die Dialyselösung beinhaltet.

Aus Figur 2 ist der hydraulische Druck im Arbeitsfluid über den Weg des Kolbens aufgetragen.

Die schraffierten Bereiche kennzeichnen das Anrampen, d.h. die Erhöhung der Kolbengeschwindigkeit, sowie das Abrampen, d.h. die Verringerung der Kolbengeschwindigkeit. In diesen mit Ü bezeichneten Bereichen, arbeiten beide Pumpen wechselseitig, so dass die schraffierten Bereiche Überlappungsbereiche des Betriebes zweier Pumpen darstellen.

Mit A ist der Kammeranfang und mit E das Kammerende der Kammer bezeichnet, in der der Kolben hin- und her bewegbar aufgenommen ist. F stellt den für die Förderung relevanten Kammerbereich dar, der variabel einstellbar ist. Mit H ist der Bereich des zu verschiebenden Hydraulikmediums gekennzeichnet.

P stellt die Pumpenplatte dar, d.h. die Membran etc., die auf die Membran der Förderkammer des Disposables einwirkt.

Erfindungsgemäß kann genau eine Pumpe eingesetzt werden. Von der Erfindung ist jedoch auch der Fall umfasst, dass zwei oder auch mehr als Pumpen vorhanden sind.

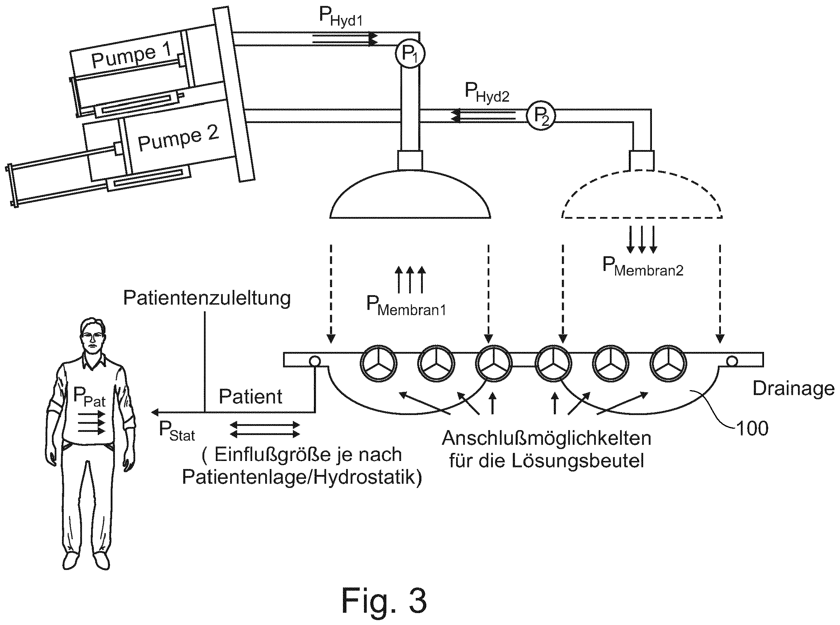

Figur 3 zeigt ein aus dem Stand der Technik bekanntes Peritonealdialysegerät bzw. -system.

Wie aus Figur 3 ersichtlich, werden üblicherweise zwei Membranpumpen (Pumpe 1, Pumpe 2) eingesetzt, die jeweils über einen Wegaufnehmer verfügen, um die Kolbenposition bestimmen zu können. Die Membranpumpen wirken auf Pumpkammern 100, mit welchen Dialysat aus entsprechend vorhandenen Dialysatbeuteln in die Bauchhöhle eines Patienten gepumpt bzw. verbrauchtes Dialysat aus der Bauchhöhle des Patienten ausgelassen wird. Um trotz der diskontinuierlich arbeitenden Membranpumpe einen konstanten Dialysatvolumenstrom zu erreichen, wird der hydraulische Druck P_{Hyd} in den Hydraulikleitungen der Membranpumpen bestimmt. Für den Fall, dass die Membranpumpen pneumatisch angetrieben werden, wird der entsprechende pneumatische Druck in den Leitungen bestimmt. Um eine Drucküberwachung zu gewährleisten, müssen die mittels der Drucksensoren gemessenen Drücke P_{Hyd} um einige Einflusswerte kompensiert werden. Hierbei handelt es sich zum einen um den jeweiligen Membrandruck P_{Membran}, d. h. den Gegendruck, der auf den gemessenen Hydraulikdruck P_{Hyd} durch die Auslenkung und Eigenspannung der Membran hervorgerufen wird. Mit zunehmender Auslenkung nimmt die Membranspannung überproportional zu, womit eine konstruktiv bedingte Geschwindigkeitsreaktion einhergeht. Dieser Gegendruck hängt von der Position der Hydraulikpumpe ab, die üblicherweise über einen Wegaufnehmer gemessen wird. Darüber hinaus wird als weitere Kompensationsgröße der Gegendruck berücksichtigt, der durch den Flusswiderstand im System, d. h. in der Pumpe und in der als Disposable ausgestalteten Pumpenkammer entsteht. Dieser zu berücksichtigende Gegendruck ist abhängig von der Geschwindigkeit im System. Schließlich ist der hydrostatische Druck P_{Stat} zu berücksichtigen, der sich aufgrund der Lage des Patienten ergibt.

## Patentansprüche

1. Pumpsystem zur Erzeugung eines Volumenstromes von Dialyselösung in einer Dialysemaschine, wobei das Pumpsystem wenigstens eine Kolbenpumpe umfasst, deren Kolben (8) mit einem Arbeitsfluid zusammenwirkt, das seinerseits eine Kraft auf ein Fördermittel, insbesondere auf eine Membran (9) ausübt, **dadurch gekennzeichnet, dass** Einstellmittel vorgesehen sind, durch die das Fördervolumen der Kolbenpumpe pro Kolbenhub verringerbar und vergrößerbar ist, wobei die Einstellmittel einen Kolbenanschlag zur Verringerung und Vergrößerung des Kolbenhubs und/oder Mittel zur Verringerung und Vergrößerung der Menge des Arbeitsfluids umfassen, wobei diese Mittel einen Vorratsbehälter zur Aufnahme des Arbeitsfluids und ein Ventil umfassen, die es ermöglichen, dass das Volumen des Arbeitsfluids verringert und vergrößert wird .

2. Pumpsystem nach Anspruch 1, **dadurch gekennzeichnet, dass** es sich bei dem Arbeitsfluid um eine Flüssigkeit oder um ein Gas handelt.

3. Pumpsystem nach Anspruch 1 oder 2, **dadurch gekennzeichnet, dass** Eingabemittel oder Berechnungsmittel vorgesehen sind, durch die das gesamte Soll-Fördervolumen oder das verbleibende Soll-Fördervolumen ab einem bestimmten Zeitpunkt oder das Soll-Fördervolumen pro Kolbenhub eingebbar bzw. bestimmbar ist, und dass die Einstellmittel mit dem Eingabemitteln oder mit den Berechnungsmitteln derart in Verbindung stehen, dass die Einstellmittel ausgebildet sind, in Abhängigkeit der in die Eingabemittel eingegebenen oder durch die Berechnungsmittel bestimmten Werte das Fördervolumen der Kolbenpumpe pro Kolbenhub einzustellen.

4. Pumpsystem nach einem der vorhergehenden Ansprüche, **dadurch gekennzeichnet, dass** die Einstellmittel derart konfiguriert sind, dass das Fördervolumen pro Kolbenhub über die gesamte Förderdauer konstant ist.

5. Pumpsystem nach einem der Ansprüche 1 bis 3, **dadurch gekennzeichnet, dass** die Einstellmittel derart konfiguriert sind, dass das Fördervolumen pro Kolbenhub über die Förderdauer veränderlich ist und insbesondere zum Ende des Fördervorgangs geringer ist als zu Beginn des Fördervorgangs.

6. Pumpsystem nach einem der vorhergehenden Ansprüche, **dadurch gekennzeichnet, dass** die Einstellmittel derart ausgebildet sind, dass die Menge des Arbeitsfluids verringerbar ist, wobei vorgesehen ist, dass die Menge des Arbeitsfluids ausgehend von einem Ausgangszustand derart verringert wird, dass der Einfluss der Membranspannung auf den Patientendruck und/oder auf den Druck des Arbeitsfluids geringer ist als im Ausgangszustand.

7. Pumpsystem nach einem der vorhergehenden Ansprüche, **dadurch gekennzeichnet, dass** die Pumpe einen Kolbenraum aufweist, der über einen Schlauch mit einer Kammer verbunden ist, die durch die Membran begrenzt wird.

8. Pumpsystem nach einem der vorhergehenden Ansprüche, **dadurch gekennzeichnet, dass** das Pumpsystem zwei Kolbenpumpen aufweist, die versetzte Arbeitszyklen aufweisen.

9. Dialysegerät, insbesondere Peritonealdialysegerät, **dadurch gekennzeichnet, dass** das Dialysegerät ein Pumpsystem nach einem der Ansprüche 1 bis 8 aufweist.

## Claims

1. Pump system for generating a volume flow of dialysis solution in a dialysis machine, wherein the pump system comprises at least one piston pump, the piston (8) of which cooperates with a working fluid which in turn exerts a force on a conveying means, in particular on a membrane (9),
**characterized in that**
setting means are provided, by which the conveying volume of the piston pump per piston stroke can be reduced and increased, wherein the setting means comprise a piston stop for reducing or increasing the piston stroke, and/or means for reducing and increasing the quantity of the working fluid, wherein these means comprise a storage tank for receiving the working fluid, and a valve, which allow for the volume of the working fluid to be reduced and increased.

2. Pump system in accordance with claim 1, **characterized in that** the working fluid is a liquid or a gas.

3. Pump system in accordance with claim 1 or 2, **characterized in that** input means or calculation means are provided, by which the total desired conveying volume or the residual desired conveying volume from a specific point in time onward or the desired conveying volume per piston stroke can be input or determined and the setting means are connected to the input means or to the calculation means such that the setting means are configured to set the conveying volume of the piston pump per piston stroke depending on the values input into the input means or determined by the calculation means.

4. Pump system in accordance with any one of the preceding claims, **characterized in that** the setting means are configured such that the conveying volume per piston stroke is constant throughout the conveying time.

5. Pump system in accordance with any one of the claims 1 to 3, **characterized in that** the setting means are configured such that the conveying volume per piston stroke is variable over the conveying time and is in particular smaller toward the end of the conveying procedure than at the start of the conveying procedure.

6. Pump system in accordance with one of the preceding claims, **characterized in that** the setting means are configured such that the quantity of the working fluid is reducible, wherein provision is made that the quantity of the working fluid is reduced, starting from an initial state, such that the influence of the membrane tension on the patient pressure and/or on the pressure of the working fluid is smaller than in the initial state.

7. Pump system in accordance with any one of the preceding claims, **characterized in that** the pump has a piston space that is in communication via a hose with a chamber that is bounded by the membrane.

8. Pump system in accordance with any one of the preceding claims, **characterized in that** the pump system comprises two piston pumps that have offset working cycles.

9. Dialysis machine, in particular a peritoneal dialysis machine, **characterized in that** the dialysis machine comprises a pump system in accordance with any one of claims 1 to 8.

## Revendications

1. Système de pompage pour la production d'un débit volumique de solution de dialyse dans une machine de dialyse, le système de pompage comprenant au moins une pompe à piston dont le piston (8) coopère avec un fluide de travail qui exerce à son tour une force sur un moyen de transport, notamment sur une membrane (9),
**caractérisé en ce**
**qu'**il est prévu des moyens de réglage grâce auxquels le volume de transport de la pompe à piston par course de piston peut être réduit et augmenté, les moyens de réglage comprenant une butée de piston pour réduire et augmenter la course du piston et/ou des moyens pour réduire et augmenter la quantité de fluide de travail, ces moyens comprenant un réservoir pour recevoir le fluide de travail et une soupape qui permettent de réduire et d'augmenter le volume du fluide de travail.

2. Système de pompage selon la revendication 1, **caractérisé en ce que** le fluide de travail consiste en un liquide ou un gaz.

3. Système de pompage selon la revendication 1 ou 2, **caractérisé en ce que** des moyens d'entrée ou des moyens de calcul sont prévus, par lesquels le volume de transport de consigne total ou le volume de transport de consigne restant à partir d'un moment déterminé ou le volume de transport de consigne par course de piston peut être entré ou déterminé, et **en ce que** les moyens de réglage sont en liaison avec moyens d'entrée ou avec les moyens de calcul de telle sorte que les moyens de réglage sont réalisés pour régler le volume de transport de la pompe à piston par course de piston en fonction des valeurs entrées dans les moyens d'entrée ou déterminées par les moyens de calcul.

4. Système de pompage selon l'une quelconque des revendications précédentes, **caractérisé en ce que** les moyens de réglage sont configurés de telle sorte que le volume de transport par course de piston est constant pendant toute la durée du transport.

5. Système de pompage selon l'une quelconque des revendications 1 à 3, **caractérisé en ce que** les moyens de réglage sont configurés de telle sorte que le volume de transport par course du piston est variable sur la durée du transport et est notamment plus faible à la fin de l'opération transport qu'au début de l'opération de transport.

6. Système de pompage selon l'une quelconque des revendications précédentes, **caractérisé en ce que** les moyens de réglage sont réalisés de telle sorte que la quantité de fluide de travail peut être réduite, il étant prévu que la quantité de fluide de travail est réduite à partir d'un état initial de telle sorte que l'influence de la tension de la membrane sur la pression du patient et/ou sur la pression du fluide de travail est inférieure à celle dans l'état initial.

7. Système de pompage selon l'une quelconque des revendications précédentes, **caractérisé en ce que** la pompe présente un espace de piston relié par un tuyau à une chambre délimitée par la membrane.

8. Système de pompage selon l'une quelconque des revendications précédentes, **caractérisé en ce que** le système de pompage présente deux pompes à piston présentant des cycles de travail décalés.

9. Appareil de dialyse, notamment appareil de dialyse péritonéale, **caractérisé en ce que** l'appareil de dialyse présente un système de pompage selon l'une quelconque des revendications 1 à 8.
